(19) Europäisches Patentamt
European Patent Office
Office européen des brevets

(11) **EP 3 874 586 B1**

(12) **EUROPÄISCHE PATENTSCHRIFT**

(45) Veröffentlichungstag und Bekanntmachung des Hinweises auf die Patenterteilung:
**12.10.2022 Patentblatt 2022/41**

(21) Anmeldenummer: **19802101.6**

(22) Anmeldetag: **04.11.2019**

(51) Internationale Patentklassifikation (IPC):
*H02K 9/19* (2006.01)  *H02K 9/10* (2006.01)
*H02K 9/24* (2006.01)  *G01N 33/00* (2006.01)
*G01M 3/22* (2006.01)  *H02K 9/08* (2006.01)

(52) Gemeinsame Patentklassifikation (CPC):
**H02K 9/24; H02K 9/197;** G01M 3/226;
G01N 33/005; G01N 33/0063; H02K 9/08

(86) Internationale Anmeldenummer:
**PCT/EP2019/080078**

(87) Internationale Veröffentlichungsnummer:
**WO 2020/126185 (25.06.2020 Gazette 2020/26)**

(54) **ANLAGE ZUR KÜHLUNG VON GENERATOREN**

EQUIPMENT FOR COOLING GENERATORS

ÉQUIPEMENT POUR REFROIDIR DES GÉNÉRATEURS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2018 CH 15812018**

(43) Veröffentlichungstag der Anmeldung:
**08.09.2021 Patentblatt 2021/36**

(73) Patentinhaber: **SvoBaTech AG**
**6300 Zug (CH)**

(72) Erfinder:
• **BAUER, Thomas**
**8006 Zürich (CH)**

• **SVOBODA, Matthias**
**5303 Würenlingen (CH)**

(74) Vertreter: **Felber, Josef et al**
**Felber & Partner AG**
**Dufourstrasse 116**
**8008 Zürich (CH)**

(56) Entgegenhaltungen:
DE-A1-102016 106 225  JP-A- H 037 040
JP-A- H0 844 081  JP-A- H05 268 750
KR-B1- 101 439 523  US-A1- 2007 277 593
US-A1- 2009 260 518  US-B1- 6 459 750

**Beschreibung**

Diese Erfindung betrifft ein System zum effizienten Kühlen der Kupferleitungen von grossen Elektro-Generatoren. Kleinste und kleine Generatoren werden mit Luft gekühlt, mittelgrosse wiederum mit Wasserstoff, und bei grossen Generatoren kommt man nicht um eine Wasserkühlung herum. Es befinden sich im Stator 1 m bis 12 m lange Stäbe, welche die Statorwicklung bilden. Diese Stäbe bestehen aus Bündeln von bis zu 40 Kupferleitern. Hydrogeneratoren weisen manchmal hunderte von Stäben auf. Manche dieser Kupferleiter sind hohl und bilden frei bleibende Kühlkanäle von einigen Quadratmillimetern Querschnitt, durch die Wasser gepumpt wird.

Etwa 50% aller Generatoren werden mit Kühlwasser mit einem Sauerstoffgehalt von > 2 ppm betrieben. Der Sauerstoff im Wasser reagiert mit den inneren Oberflächen dieser Kupferhohlleiter von Grossgeneratoren und es wird an den Wänden der Kühlkanäle eine Schicht von Kupfer-Oxid gebildet. Dabei handelt es sich um eine sich fliessend neu bildende Schicht, während ebenso fliessend ein Teil der Schicht durch das vorbeiströmende Wasser abgebaut wird. Im Idealfall soll die Schicht über die Zeit gleichstark bleiben, das heisst der Schichtaufbau und Schichtabbau sollen sich in einem Gleichgewicht halten.

Grossgeneratoren zur Stromerzeugung werden oft mit direkter Wasserkühlung in der Kupferwicklung ausgestattet. Das Kühlwasser zirkuliert in einem geschlossenen Kreislauf mit einer kontinuierlichen Demineralisierung in einem partiellen Bypassstrom. Dies garantiert Reinstwasser-Bedingungen, um die Interaktionen mit den Systemmaterialien zu minimieren. Insbesondere die Kupferoberflächen in der Statorwicklung, welche direkt dem Kühlwasser exponiert sind, müssen möglichst stabil gehalten werden. Dazu ist die Wasserchemie sorgfältig zu kontrollieren. Neben löslichen Verunreinigungen, welche mittels des Ionentauschers im Demineralisations-Bypass kontrolliert werden, muss der Sauerstoffgehalt entweder sehr tief oder sehr hoch gehalten werden. Betreibt man die Kühlung mit viel $O_2$ im Kühlwasser, mit über 2 ppm, so bildet sich eine stabile CuO-Schicht, während bei niedrigem $O_2$-Gehalt, unter 20 ppb versucht wird, die Kupferoxidbildung zu minimieren. In einem Bereich des $O_2$-Gehaltes im Kühlwasser zwischen 20 ppb und 2 ppm bildet sich eine instabile Kupferoxid Mischung. Man versucht insbesondere, einen $O_2$-Anteil zwischen 200 ppb, und 1000 ppb zu vermeiden, weil bei diesem $O_2$-Anteil die CuO-Schicht am instabilsten ist. Vielmehr versucht man daher, mit dem $O_2$-Anteil entweder gegen 0 ppb zu gehen, oder aber mit einem $O_2$-Anteil von über 2000 ppb zu fahren. Bei hohem $O_2$-Anteil bildet sich eine stabile Schicht von hauptsächlich CuO auf den Innenwänden der Kühlkanäle. Ein weiterer zu berücksichtigender Aspekt ist der $CO_2$-Gehalt im Kühlwasser, denn $CO_2$ erniedrigt den pH-Wert des Kühlwassers und dieses greift dann die CuO-Schicht an, das heisst diese Schicht wird löslich und instabil, was im schlimmsten Fall zu Verstopfungen der Kühlkanäle führt.

Um den Sauerstoffgehalt zu kontrollieren, kann Gas in das System eingeblasen werden. Für Systeme mit tiefem Sauerstoffgehalt wäre dies üblicherweise Stickstoff $N_2$, für Systeme mit hohem Sauerstoffgehalt üblicherweise Luft. Meist wird in Fällen von tiefem Sauerstoffgehalt kein Gas eingeblasen, aber ein Schutzgas im Tank gehalten. Wenn bei hohem Sauerstoffgehalt Luft eingeblasen wird, so bringt das den Nachteil mit sich, dass damit auch $CO_2$ in das System eingebracht wird, welches wie erwähnt das Wasser ansäuert und damit die Oxidschicht auf den Kupferoberflächen destabilisiert. Um dies zu minimieren sollte das $CO_2$ aus der eingeblasenen Luft vorgängig entfernt werden.

Zusätzlich zur Wasserkühlung werden Grossgeneratoren auch mit Wasserstoffgas gekühlt, welches im Innern des Generatorgehäuses zirkuliert. Wasserstoff-Moleküle diffundieren aber unvermeidlich durch die Teflonschläuche in das Kühlwassersystem des Stators. Wenn jedoch ein Leck in den Kupferstäben des Stators oder den Verbindungen vorliegt, strömen grössere Mengen an Wasserstoff in das Kühlwasser, weil der Wasserstoffdruck im Inneren des Generatorgehäuses absichtlich höher gehalten wird als der Kühlwasserdruck. Wenn $H_2$ ins Kühlwasser eindringt, ist das nämlich weit weniger gefährlich als wenn umgekehrt Kühlwasser in das Generatorgehäuse gelangen würde.

Das Kühlwassersystem muss einen Mechanismus zur Entgasung des Wassers aufweisen. Oft wird diese Aufgabe mit einem Tank im Hauptstrom gelöst, welcher eine Entlüftungsleitung zur Atmosphäre hat. Durch diese Entlüftung entweicht auch der Wasserstoff, welcher wie oben erklärt unvermeidlich in geringen Mengen ins System gelangt.

In der praktischen Ausführung ist ein mit Kühlwasser gefüllter Speichertank vorhanden, und von unten wird Luft in denselben eingepumpt. Der Gehalt eines gelösten Gases im Wasser richtet sich im Gleichgewicht nach dessen Partialdruck im Gasraum des Speichertanks. Im Folgenden werden nur noch Systeme in Betracht gezogen, die mit einem hohen Sauerstoffgehalt im Kühlwasser gefahren werden. Man sättigt das Kühlwasser mit $CO_2$-freier Luft zum Anreichern mit $O_2$. Ein Anteil des $O_2$ reagiert mit dem Kupfer der Innenwände der Kühlkanäle und bildet auf denselben eine Kupfer-Oxid-Schicht. In dem Wasser, welches den Generator verlässt, ist auch wenig Wasserstoff enthalten, welcher wiederum im Tank entsprechend seines Partialdrucks ausgast. Die Abluft mit einem ganz geringen Anteil an $H_2$ wird aus dem Speichertank über eine Entlüftungs-Steigleitung ins Freie abgelassen. Die Sauerstoff-Anreicherung dient dazu, konstant einen Sauerstoffgehalt von > 2000 ppb zu erhalten, um die Kupfer-Oxide-Schicht möglichst stabil zu halten.

Nach der herkömmlichen Lehre hält man die Luftinjektionsrate bewusst gering, auf <0.15 CFM = 4.25 L/min, weil man davon ausgeht, dass dann ein Wasserstoff-Anteil in der Luft bzw. die Wasserstoffkonzentration in diesem Luftanteil einfacher messbar sei als in einem grossen Luftvolumen, in welchem der Wasserstoff nur einen ganz geringen und vermeintlich schwierig nachzuweisenden Anteil einnähme.

Die von den Generatorherstellern gelieferten und installierten teuren Messgeräte für die Bestimmung des $H_2$-Gehalts in der Abluft werden in der Praxis meist nicht beachtet und sie werden weitgehend sich selbst überlassen. Es gibt noch kein Gerät, welches regelmässig den $CO_2$-Gehalt in der Luft, welche injiziert wird, misst, sondern es wird einfach davon ausgegangen, dass $CO_2$ wirksam entfernt wird. $CO_2$ in der eingeblasenen Luft greift aber die Kupfer-Oxid Schicht an den Kühlkanal-Innenwänden wie erwähnt an, und somit ist es erstrebenswert, den $CO_2$ Gehalt in der Luft genau zu kennen, und um zu erkennen, wenn die $CO_2$-Entfernung fehlerhaft ist.

In der Praxis wird von den Herstellern der Generatoren angegeben, in der eingeblasenen Luft trete kein $CO_2$ auf. Das stimmt bei Inbetriebnahme der Generatoren in der Regel, aber über einige Betriebsjahre hinaus trifft das nicht mehr immer zu und es können erhebliche $CO_2$-Anteile im eintretenden Luftstrom gemessen werden. Weil zu jenem Zeitpunkt möglicherweise anfänglich vorhandene Garantien abgelaufen sind, dann ist der Kraftwerkbetreiber mit dem Problem konfrontiert und damit allein gelassen, dass sich die Kühlkanäle zusetzen. Dieses Problem äussert sich in der Regel erst dann, wenn die Kühlfunktion unversehens beeinträchtigt ist und die Temperaturen ansteigen oder sogar einen unzulässigen Grenzwert übersteigen. Die Kraftwerkbetreiber wünschen sich Abhilfe, um dieses Problem ein- für allemal in den Griff zu bekommen.

Im Stand der Technik ist besonders auf US 2007/0277593 A1 hinzuweisen. Dieses Patent ist eine Aktualisierung eines älteren Patentes zum alten Stator-Leckage-Monitoring mit als wichtigste Änderungen der Verringerung der Lufteinblasrate auf <0,15 CFM (=4,25 L/min), was zu einer Erhöhung der Genauigkeit des Überwachungssystems auf etwa 0,2 CFD Wasserstoffleckrate führt. Der größte Nachteil ist aber, dass bei dieser geringen Lufteinblasung unter normalen Betriebsbedingungen explosive Gaskonzentrationen, das heisst ein Gemisch von Wasserstoff und Luft im Tank und in der zur Atmosphäre offenen Entlüftungsleitung vorkommen können. Es gibt mehr als 1'000 Generatoren mit hohem Sauerstoffgehalt im Kühlwasser, die alle über eine korrekte $CO_2$-freie Lufteinblasung verfügen sollten. Es sind Hunderte von Systemen in Betrieb, teils schon seit Jahrzehnten, und an diesen wird die Wartung oft zuwenig konsequent durchgeführt. Weiter sind für den Stand der Technik folgende Dokumente relevant:

D1: JP H03 7040 A (TOSHIBA CORP) vom 14. Januar 1991,
D2: US 6 459 750 B1 (ITO° KAZUTOSHI [JP] ET AL) vom 1. Oktober 2002,
D3: JP H05 268750 A (TOSHIBA CORP) vom 15. Oktober 1993,
D4: US 2007/277593 A1 (SALEM SAMEH [US] ET AL) vom 6. Dezember 2007,
D5: JP H08 44081 A (KONISHIROKU PHOTO IND( vom 16. Februar 1996,
D6: KR 101 439 523 B1 (ANYTECH [KR]() vom 15. September 2014,
D7: US 2009/260518 A1 (WRIGHT ANDREW DAVID [GB] ET AL. vom 22. Oktober 2009,
D8: DE 10 2016 106225 A1 (KRINNER DRUCKLUFTTECHNIK GMBH [DE] vom 5. Oktober 2017.

Das Dokument D2 offenbart ein Verfahren zum Kühlen (Spalte 2, Zeilen 55-67) der in grossen Elektro-Generatoren vorhandenen Kühlwasser leitendenden Kupferleitungen (6, Spalte 6, Zeilen 47-54). Je nach Systemparameter (Sauerstoffgehalt des Kühlwassers, Spalte 2, Zeile 58) wird gezielt $CO_2$-freie (Spalte 2, Zeilen 55-67) öl- und staubfreie Luft in einen Kühlkreis eingepumpt (Spalte 2, Zeilen 55-67). Somit wird stets ausserhalb des kritischen Luftmengenanteils für den im Kühlkreislauf immer enthaltenen Wasserstoff $H_2$, das heisst unterhalb von 4-75% $H_2$ in der Luft, und somit ausserhalb des gefährlichen Mengenverhältnisses zur Vermeidung von Wasserstoff-Explosionen, (Spalte 2, Zeilen 4-9 sowie Spalte 6, Zeilen 42-46) Luft eingepumpt, wobei der in der eingepumpten Luft enthaltene Sauerstoff im Stator-Kühlwasser-System mit dem Kupfer der Kühlkanäle reagiert und sich an deren Innenwänden eine Schicht Kupfer-Oxid bildet, welche zum Schutz der Kupferoberflächen des Stators vor Korrosion (Spalte 3, Zeile 45 Spalte 4, Zeile 22) wirkt. D2 offenbart aber nicht, dass das jeweilige Gas mit einer Injektionsrate von mehr als 0.15 cfm in den Kühlkreis eingepumpt wird. Jedoch ist eine Luftinjektion, mit der genügend Sauerstoff injiziert wird, mit 4000 ppb im Kühlwasser aus D2 bekannt (4ppm = 4000ppb, Spalte 8, Zeilen 40-46). Dies ist doppelt so hoch wie die untere Konzentrationsgrenze der Anwendung von 2000 ppb Sauerstoff. Daraus lässt sich aber keine Untergrenze der Injektionsrate ableiten, die größer ist als die Untergrenze von 0.15 cfm. Nach D2 wird die Konzentration des Sauerstoffs im Wasser durch die eingeblasene Luft gesteuert wird (Spalte 8, Zeilen 40-46). D2 offenbart jedoch nicht, dass die Sauerstoffkonzentration im Wasser durch den Sauerstoffgehalt des Gasraums im Tank kontrolliert wird. Auch wird nicht offenbart, dass der $CO_2$ Gehalt in der zugeführten $CO_2$-freien öl- und staubfreien Zuluft laufend gemessen und überwacht wird. Hingegen werden in Spalte 4, Zeilen 5-9 sehr spezifische und niedrige Werte für $CO_2$ angegeben. Allerdings wird eine Überwachung nicht gemacht. Eine Messung für dekarbonisierte Luft ist in der Technik zwar üblich (z.B. Absatz 41 des 05, Absatz 11 des D6, Absätze 35-37 des D7, und Absatz 28 des D8). Ein kritischer $H_2$-Gehaltes von >2% ist weder von D1 noch von D3 bekannt.

Die Aufgabe der vorliegenden Erfindung ist es deshalb, eine Anlage zur effizienteren und sichereren Kühlung von elektrischen Gross-Generatoren anzugeben, mit welcher eine sichere Vermeidung von explosiven Sauerstoff-Wasserstoff-Mischungen erreicht werden kann. Die Lufteinblasung soll insgesamt auch kostengünstiger und sicher über-wachbar sein und die $CO_2$-Konzentration ins Kühlwasser eingeleiteten Luft soll jederzeit messbar und unter einem bestimmten Grenzwert haltbar sein.

Die Lösung für diese Aufgabe besteht in einer Anlage zum Kühlen von in grossen, mit Wasserstoffgas gekühlten Elektro-Generatoren vorhandenen, Kühlwasser leitenden Kupferleitungen, mit welcher Anlage gezielt je nach dem Sauerstoffgehalt des Kühlwassers bei hohem Sauerstoffgehalt eine Mindestmenge an $CO_2$-freier Luft oder bei tiefem Sauerstoffgehalt reiner Stickstoff $N_2$ in den Kühlkreis einpumpbar ist nach dem Oberbegriff und mit den kennzeichnenden Merkmalen des Patentanspruchs 1. Es wird mit der Anlage gezielt je nach Systemparameter entweder $CO_2$-freie Luft oder reiner Stickstoff $N_2$ in den Kühlkreis eingepumpt wird, wobei sie an Stator-Kühlwassersystemen mit niedrigem Sauerstoffgehalt einsetzbar ist, ohne $CO_2$-Entfernung, indem anstelle von Luft reiner Stickstoff $N_2$ in das Stator-Kühlwassersystemen einblasbar ist und die Wasserstoffleckage messbar und anzeigbar ist. Der $H_2$-Gehalt in der abgeführten Abluft wird laufend gemessen und überwacht, und bei Messung eines kritischen $H_2$-Gehaltes in der Abluft von >2% wird ein akustischer oder optischer Alarm wegen einer Annäherung an das explosive Mengenverhältnis ausgelöst. Weiter wird die Aufgabe gelöst von einer Anlage mit dem Merkmalen des Patentanspruchs 4.

Mit dieser Anlage wird bezweckt, dass einerseits das entsprechende Gas ins System eingebracht wird, und andererseits die Wasserstoffleckage laufend gemessen und überwacht wird. Um dieses zu bewerkstelligen, wird Folgendes durchgeführt:

1. Für Systeme mit hohem Sauerstoffgehalt wird $CO_2$-freie Luft injiziert, für Systeme mit tiefem Sauerstoffgehalt wird Stickstoff $N_2$ injiziert.

2. Über ein Monitoring-System wird die Wasserstoffkonzentration an der Entlüftungsleitung laufend gemessen und überwacht.

3. Mit einer elektronischen Steuereinheit als Kontrollbox und einem Display wird anhand der gemessenen Daten des Luftstroms und der Wasserstoffkonzentration eine Wasserstoffleckage erkannt und es werden die Schlüsseldaten angezeigt.

4. Über ein Monitoring-System wird die $CO_2$-Konzentration der injizierten Luft gemessen und an der Steuereinheit dargestellt.

5. Optional können zusätzliche Instrumente angeschlossen sein, um weitere Chemieparameter des Kühlwassersystems zu messen und einzuhalten.

Die Anlage zum Anschluss an ein Stator-Kühlwasser-System weist einen Stator-Kühlwassertank mit abgehender Leitung und Steigrohr auf, wobei die abgehende Leitung das Kühlwasser durch Pumpen, Wärmetauscher, einen Filter und hernach wahlweise durch einen Ionentauscher zurück in den Stator-Kühlwassertank führt oder in die Kühlkanäle einer Statorwicklung in einem Generator, und ist *dadurch gekennzeichnet,* dass sie ein Luftinjektions-System mit Kontrollsystem einschliesst, mit Messinstrumenten für das Messen von $CO_2$ in der Zuluft und $H_2$ in der Abluft, sowie mit einer Steuereinheit mit Bedienelementen, wobei für die $CO_2$-Konzentration wie auch für die $H_2$-Konzentration Grenzwerte eingebbar sind, bei deren Überschreiten ein Alarm abgebbar ist oder Massnahmen automatisch einleitbar sind.

Eine solche Anlage wird in den Figuren anhand einer schematischen Darstellung gezeigt. Ihre Komponenten und ihre Funktion sowie das damit durchgeführte Verfahren werden im Einzelnen beschrieben und erläutert.

Es zeigt:

Figur 1: Ein Schema einer konventionellen Generator-Kühlung mit ihren Komponenten, um später den Unterschied der erfindungsgemässen Anlage dazu und des damit betriebenen Verfahrens klarer aufzeigen zu können;

Figur 2: Das Schema eines Generators mit Kupferstäben mit deren Kühlkreislauf und dem Speichertank im grundsätzlichen Aufbau;

Figur 3: Das Schema eines Generators mit Kupferstäben mit deren Kühlkreislauf und dem Speichertank, der Entlüftungs-Steigleitung und der angeschlossenen erfindungsgemässen Anlage für die Durchführung des Verfahrens;

Figur 4: Ein Schema eines $CO_2$-Abscheiders für Luft.

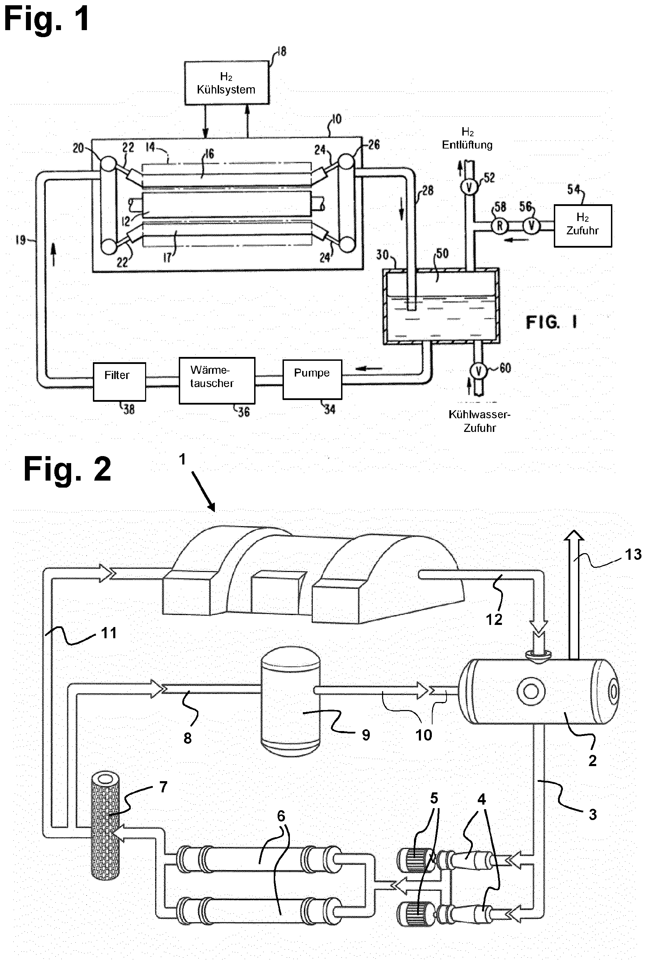

Zunächst zeigt das Schema nach **Figur** 1 eine konventionelle Kühlung eines Generators mittels Wasser. Im Grundsatz geht es um die Kühlung der Kupferstäbe 16, 17 in der Figur 1. Diese Kupferstäbe sind der Länge nach von Kühlkanälen durchsetzt. Leitungen aus Teflon sind als Zuleitungen 22 und als Ableitungen 24 an diese Kühlkanäle 16, 17 angeschlossen. Es ist ein Wassertank 30 vorhanden, welcher im Bild zu ca. 2/3 mit Wasser gefüllt ist, während oberhalb des Wasserspiegels Raum 50 für Gas bzw. Luft vorhanden ist. Über ein Ventil 60 kann Kühlwasser dem Tank zugeführt werden. Das Kühlwasser fliesst nach unten ab durch die Leitung 19 und fliesst dann durch eine Pumpe 34, einen Wärmetauscher 36 und einen Filter 38, wonach es über den Verteiler 20 und die daran angeschlossenen Zuleitungen 22 in die Kühlkanäle 16, 17 eingespiesen wird. Nach Wärmeaufnahme fliesst es auf der anderen Seite der Kühlkanäle über die Ableitungen 24 und einen Sammler 26 über die Leitung 28 zurück in den Tank 30. Der Generator bzw. dessen Kupferstäbe werden von aussen mit Wasserstoffgas $H_2$ umspült. Dabei diffundiert unweigerlich ein geringer Anteil Wasserstoff $H_2$ durch die Teflonschläuche 22 und 24 hindurch ins Innere des Kühlwassers. Dieser Wasserstoff wird dann vom Kühlwasser mitgeführt und gelangt in den Tank 30 und entweicht aus diesem über die Steigleitung. Zusätzlich wird nach dem Stand der Technik Luft ins Kühlwasser eingepumpt. Diesen Luftanteil hält man bewusst gering, sodass der Volumenstrom jedenfalls unterhalb von 4.25 l/min bleibt. Nach der gängigen Lehrmeinung stelle nur ein so geringer Luftanteil sicher, dass man den Wasserstoffgehalt im Kühlwasser zuverlässig überwachen könne.

Die **Figur** 2 zeigt schematisch einen Aufbau eines Kühlsystems für einen wassergekühlten Generator 1. Aus einem Wassertank 2 wird über die Leitung 3 durch die Pumpen 4 und ihre zugehörigen Elektromotoren 5 Wasser durch einen Wärmetauscher 6 gepumpt, in welchem Wärme aus dem Wasser abgezogen wird, sodass kühles Wasser hernach durch einen Filter 7 strömt. Nach dem Filter 7 zweigt eine Wasserleitung 8 in einen Ionentauscher 9 ab, welches Wasser hernach über die Rücklaufleitung 10 in den Wassertank 2 zurückgeführt wird. Dieser Nebenstrom ist üblicherweise konstant und er dient dazu, mögliche Verunreinigungen, unter anderem $CO_2$, Kupferoxide, etc. zu entfernen. Weil der Generator im Innern seines Gehäuses mit Wasserstoffgas umspült ist, und unweigerlich ein geringer Anteil Wasserstoff durch die Teflonschläuche ins Kühlwasser eindringt, wird dieser zusammen mit dem Kühlwasser, welches im Innern des Generators Wärme aufnahm, zurück in den Tank 2 geführt. Von dort entweicht der Wasserstoff dem Tank 2 über eine lange Steigleitung 13 in die Atmosphäre.

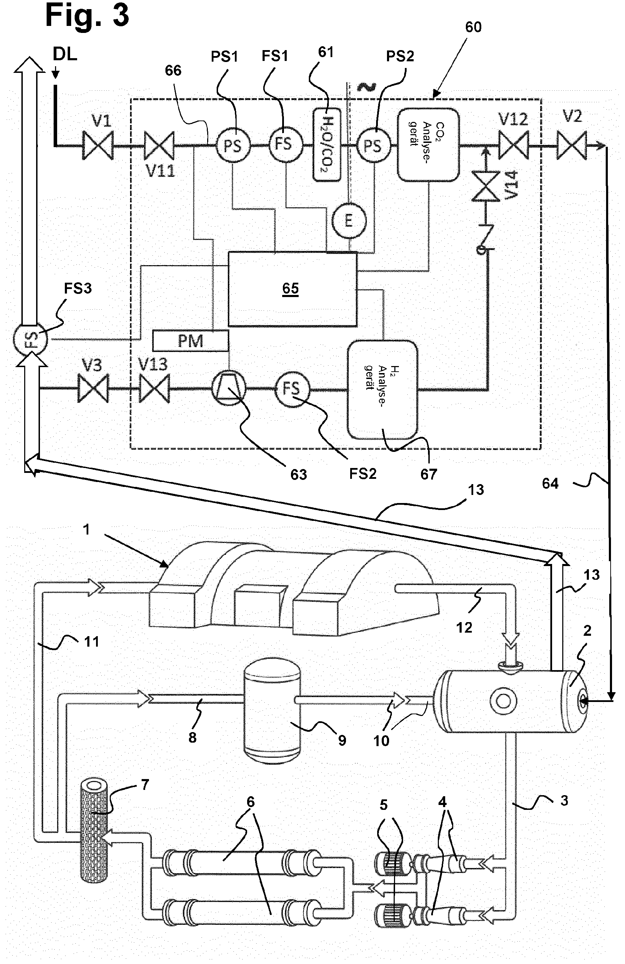

Die **Figur** 3 zeigt die wesentlichen Komponenten der erfindungsgemässen Anlage zum Durchführen des Verfahrens, zusammengeschaltet mit einem Kühlsystem wie eben vorgestellt. Aus dem Kühlwassertank 2 führt eine Steigleitung 13 nach oben. Durch diese wird aus dem Kühlwasser ausgeschiedener Wasserstoff an die Atmosphäre abgegeben. Gemäss dem neuen Konzept wird mit einem InjektionsModul gearbeitet, das in **Figur** 3 mit einer gestrichelten Linie umfasst ist und insgesamt mit 60 bezeichnet ist. Kernstück dieses Konzeptes ist es, dass dem Kühlkreisluft $CO_2$-freie Luft zugeführt wird, und zwar in einem wesentlich höheren Mass als bisher praktiziert, nämlich mit einer Injektionsrate von mehr als 70 cm$^3$/sBisher wurde diese Injektionsrate empirisch gewählt und eher tief gehalten. Druckluft DL, vom Kraftwerk bereitgestellt, wird über ein Ventil V1, das Kraftwerk-seitig vorhanden ist, über ein zur Anlage gehöriges weiteres V11 in die Anlage gepumpt. Mit einem Drucksensor PS1 für einen Bereich 0-100 psi, mit einem typischen Betriebsregime von 70 psi Eingang und 50 psi Ausgang, wird laufend der Druck der angesaugten Luft gemessen, und mit einem Durchflussmesser FS1 von 0-10 l/min wird der Luftdurchfluss gemessen.

Der Wasser- und $CO_2$-Anteil wird in einem Aggregat 61 abgeschieden. Es folgt ein weiterer Drucksensor PS2. Dann strömt die Luft durch ein $CO_2$ Analysegerät. Dieses ist in der Lage, die $CO_2$-Konzentration von 0-1000 ppm anzuzeigen. Dieses $CO_2$-Analysegerät 62 überprüft die Reinigungswirkung des $CO_2$-Entferners, um $CO_2$-freie Luft zu garantieren. Die von $CO_2$ entlastete Kühlluft geht dann via das Anlagen-seitige Ventil V12 und dann über ein Generatorseitiges Ventil V2 über die Leitung 64 zum Kühlwassertank 2. Aus diesem wird das mit $CO_2$-freier Luft angereicherte Kühlwasser schliesslich in die Kühlkanäle in den Kupferstäben des Generators 1 gepumpt. Die injizierte Luft dient nicht nur zur Anreicherung des Kühlwassers mit Sauerstoff, sondern zusätzlich wird auch noch ein Teil des Wasserstoff $H_2$ aus dem Tank 2 entfernt. Dieses Wasserstoff-Luft Gemisch wird über das Steigrohr 13 abgelassen. Aus der Steigleitung 13 wird Gas entnommen und durch das Generatorseitige Ventil V3 und das Anlagen-seitige Ventil V13 über das Pumpenrad 63 und über einen Durchfluss-Sensor FS2 zu einem $H_2$-Analysegerät 67 gefördert. Die Durchflussmenge wird hier niedriger als in der Luftinjektion gehalten. Das System kann auch für Systeme mit niedrigem Sauerstoffgehalt eingesetzt werden, in einer vereinfachten Variante auch ohne $CO_2$-Entfernung, indem anstelle von Luft reiner Stickstoff $N_2$ eingeblasen wird.

Wie aus **Figur** 3 weiter ersichtlich, führen elektrische Leitungen von allen Anlagekomponenten, nämlich vom Drucksensor PS1, vom Durchflussmesser FS1, vom Aggregat 61 für die $H_2O/CO_2$ Überprüfung, vom Ein/Aus-Schalter E, vom zweiten Drucksensor PS2, vom $CO_2$-Analysegerät 62, vom $H_2$-Analysegerät 67, vom zweiten Durchflussmesser FS2, von der Pumpe PM für den Durchfluss des Kühlwassers und vom Gas-Durchflussmesser FS3 im Steigrohr 13 zu einer zentralen elektronischen Steuereinheit 65, welche die Daten von all diesen Komponenten verarbeitet und an einem zugehörigen Display zur Anzeige bringt, wobei an dieser elektronischen Steuereinheit 65 über ein zugehöriges Bedienfeld je nach Bedarf gewünschte Parameter zuhanden der Steuereinheit 65 eingegeben werden können, zum Beispiel systemtypische Grenzwerte. Die eingezeichneten Ventile V11 bis V14 können wahlweise elektronisch steuerbare Magnetventile sein, um das ganze Injektionsmodul 60 bedarfsweise via die zentrale elektronische Steuereinheit 65 an ein Kühlsystem an- oder abzukoppeln.

Neu wird nun also Luft nach diesem vorliegenden Kühlkonzept in wesentlich grösserem Umfang in das Kühlwasser eingepumpt, um einen Volumenstrom von jedenfalls mehr als 5 l/min zu erreichen. Der Volumenstrom der von $CO_2$ gereinigten Luft, die eingeblasen wird, ist im Rahmen eines noch akzeptabel grossen Wasserstofflecks nämlich genügend hoch, um ein explosives Gemisch mit Wasserstoff sicher zu vermeiden. Ist das Leck grösser, muss der Generator 1 sowieso zur Reparatur ausgeschaltet werden. Dieser grosse Volumenstrom an Luft unterscheidet sich von den gängigen Lösungen, die mit einem Volumenstrom von <4.25 l/min arbeiten. Der genügend genaue Nachweis des $H_2$-Anteils in der Luft für die kontinuierliche Überwachung ist mit neuen $H_2$ Messgeräten auch bei kleinsten Konzentra-

tionen von $H_2$ in der Luft möglich. Dadurch kann man sicherstellen, dass kein explosives Gasgemisch im Kühlwassertank 2 und der Steigleitung 13 vorhanden ist.

Bei mehreren chemischen Reinigungen bei Systemen mit hohem Sauerstoffgehalt war eines der Probleme das Eindringen von $CO_2$ in das Stator-Cooling-Water-System SCWS durch das Stator-Leckage-Monitoring-System SLMS. Das SLMS ist mit einem $CO_2$-Entferner ausgestattet, aber dieser leidet in der Praxis oft an Nichtfunktion oder Fehlfunktion und bietet unzutreffende Rückmeldungen. Daher besticht eine wirklich funktionierende $CO_2$-freie Lufteinblasung, vorzugsweise in Kombination mit einem ebenfalls wirklich funktionierenden Wasserstoff-Leckage-Monitoring-System, denn oftmals arbeiten die derzeit installierten Monitoring-Systeme ebenfalls nicht zufriedenstellend.

Alle Daten aus dem Lufteinlasssystem sowie dem $H_2$-Analysator 67 gehen wie schon erwähnt in eine elektrische Steuereinheit 65 in einem Schaltkasten. Damit ist eine Kontroll-Box gebildet. In dieser werden wenigstens die Wasserstoff-Leckrate berechnet und die wichtigsten Parameter und Alarme werden zur Anzeige gebracht. Die Daten Speicherung kann auf einem Festspeicher oder USB-Stick sowie über eine mögliche Online-Datenübertragung an einen Server eingerichtet werden. Die Wasserstoffleckrate kann nach folgender Formel aus der Luftmenge berechnet werden, die in das System eingespritzt wird, und der Wasserstoffkonzentration in der Luft, welche das System verlässt.

$$H2\,(Leckage) = \frac{(-\text{Luft (eingeblasen)} * \text{H2 (gemessen)})}{(\text{H2 (gemessen)} - \text{H2 (Reinheit)})}$$

| | |
|---|---|
| $H_2$ (Leckage) | $H_2$ Leckage-Rate |
| Luft (eingeblasen) | Luft-Injektionsrate |
| $H_2$ (gemessen) | gemessene Konzentration von $H_2$ |
| $H_2$ (Reinheit) | $H_2$-Reinheit des Kühlgases (typischerweise 95-99%) |

Das Besondere am vorliegenden Kühlkonzept ist es, dass mit einer bewusst höheren Lufteinblasrate von > 0,15 cfm bzw. 70 cm$^3$/s gearbeitet wird, und das Risiko explosiver Gase in der Lüftungsleitung sowie dem Expansionstank verhindert wird, was ein wesentliches Sicherheitsmerkmal darstellt. Für die Berechnung der Lufteinblas-Menge wird nach dieser neuen Formel auch die Wasserstoffreinheit des Wasserstoffkühlgases als Messgrösse mit einbezogen.

Die Anlage kann modular aufgebaut sein, um entweder nur das Lufteinlasssystem oder auch nur die Wasserstoffleckage-Überwachung bereitzustellen. Je nach Ausführung muss die Wasserstoffleckage-Überwachung über ein Lufteinlasssystem verfügen. Sie beinhaltet immer den Schaltkasten mit einer elektronischen Steuereinheit 65 als Kontroll-Box, mit allen notwendigen Anschlüsse für zukünftige Erweiterungen, wenn nur eines der beiden modularen Systeme installiert ist. Abhängig vom Design des Kühlwassersystems liegen bis zu 20 Cubic-Feet bzw. 0.5663 m$^3$ $H_2$ Leckagen vor, ohne dass es zu einer explosiven Mischung im Generator kommt. Dabei handelt es sich schon um mittlere Leckagen, die naheliegend repariert werden sollten bzw. müssen. Beispielsweise Richtwerte und die daraus zu implizierenden Anzeigen sind folgende:

- Dichtes System: 0,2 Cubic-Feet = 0.005663 m$^3$/Tag: 0,08% $H_2$ in Luft
- Normales System: 1 Cubic-Feet = 0.02832 m$^3$/Tag: 0,38% $H_2$ in Luft
- 5 Cubic-Feet = 0.1416 m$^3$/Tag: 1,91% $H_2$ in Luft: Es erfolgt ein akustischer und optischer ALARM wegen des Vorhandenseins potenziell explosiver Gasgemische! Ab 4% ist ein Gemisch explosiv und somit gefährlich. Mit einer Alarmschwelle von 1.91% $H_2$ in der Luft ist besteht eine Sicherheitsmarge von 100%.

- Mittlere Leckage: 20 Cubic-Feet = 0.5663 m$^3$/Tag: 7,63% $H_2$ in Luft
- Starke Leckage: >50 Cubic-Feet = 1.416 m$^3$/Tag: >19,07 % $H_2$ in Luft

Die zur Anlage gehörige elektronische Steuereinheit 65 schliesst folgende Funktionen ein, die über ein Bedienfeld abrufbar und am zugehörigen Display anzeigbar sind.

- Anzeige des Lufteinlassstroms
- Alarm oder Anzeige der $CO_2$-Konzentration der Lufteinblasung
- Anzeige der Wasserstoffkonzentration in der Entlüftungsleitung
- Berechnung und Anzeige der Wasserstoffleckrate
- Datenspeicherung über USB
- Potenziell erweiterbar zur drahtlosen Datenübertragung zu einem Server
- Löst akustischen und optischen Alarm aus bei:

hoher $CO_2$-Injektion in das Stator-Cooling-Water-System SCWS Fehlfunktion der Lufteinblasung Wasserstoff-Gehalt erreicht >2%

In Zukunft kann die Anlage für dieses Stator-Kühlwassersystem (Stator Cooling Water System SCWS) mit einem Chemieinstrumentierungs-Modul kombiniert werden.

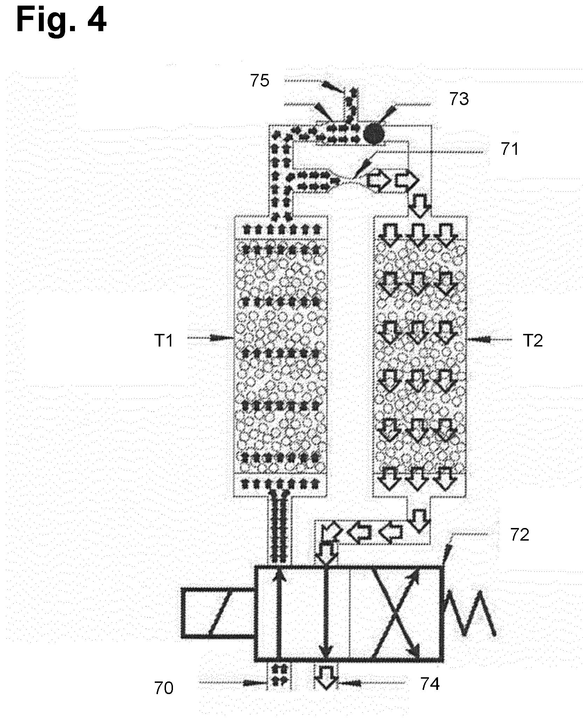

In **Figur 4** ist noch ein Schema eines $CO_2$-Abscheiders für Luft dargestellt um dessen Funktion zu erläutern. Die zu reinigende Luft strömt durch den Einlass 70 in die Absorber-Kolonne T1. Die Luft, das heisst die mit $CO_2$ und $H_2O$ belastete Luft fliesst dann von unten nach oben durch diese Absorber-Kolonne T1. Typischerweise wird mehr als 50% der in dieser Absorber-Kolonne T1 gereinigten Luft in der Regenerations-Kolonne T2 für deren Regeneration verwendet, während die restliche Luft $CO_2$-frei ist und über das Rückschlagventil 71 und den Anschluss 75 abgezogen werden kann und für eine Verwendung zur Verfügung steht. Ein Solenoid-VierwegeVentil 72 wird periodisch von Absorber-Kolonne T1 zur Regenerations-Kolonne T2 und umgekehrt gewechselt. Die Regeneration kann durch eine Heizung unterstützt werden, zum Heizen der Regenerations-Kolonne T2 oder direkt des Regenerationsgases, indem die Kolonne T2 unter einen Unterdruck gesetzt wird, und durch Entlassen von Regenerationsgas auf Umgebungsdruck, während die Absorption in der Kolonne T1 auf einem erhöhten Druckniveau erfolgt.

## Patentansprüche

1.  Anlage zum Kühlen von in grossen, mit Wasserstoffgas gekühlten Elektro-Generatoren vorhandenen, Kühlwasser leitendenden Kupferleitungen, mit welcher Anlage gezielt je nach dem Sauerstoffgehalt des Kühlwassers bei hohem Sauerstoffgehalt eine Mindestmenge an $CO_2$-freier Luft oder bei tiefem Sauerstoffgehalt reiner Stickstoff $N_2$ in den Kühlwasser-Kühlkreis einpumpbar ist, und womit der kritische Luftmengenanteil für den im Kühlkreislauf enthaltenen Wasserstoff $H_2$ ausserhalb des Bereichs von 4-75% $H_2$ in der im Kühlwasser enthaltenen Luft und somit ausserhalb des gefährlichen Mengenverhältnisses haltbar ist, mit einem Anschluss an ein Stator-Kühlwasser-System, wobei die Anlage einen Stator-Kühlwassertank (2) mit abgehender Leitung (3) und Steigrohr (13) aufweist, und wobei die abgehende Leitung (3) das Kühlwasser durch Pumpen (4), Wärmetauscher (6), einen Filter (7) und hernach wahlweise durch einen Ionentauscher (9) zurück in den Stator-Kühlwassertank (2) führt oder in die Kühlkanäle einer Statorwicklung in einem Generator (1), und wobei die Anlage ein Luftinjektions-System (60) mit Kontrollsystem einschliesst, mit Messinstrumenten für das kontinuierliche Messen von $CO_2$ in der zugeführten Zuluft und $H_2$ in der abgeführten Abluft, sowie eine Steuereinheit (65) mit Bedienelementen, wobei für die $CO_2$-Konzentration wie auch für die $H_2$-Konzentration Grenzwerte eingebbar sind, bei deren Überschreiten ein Alarm abgebbar ist oder Massnahmen automatisch einleitbar sind, wobei das Luftinjektions-System (60) mit einem anlagenseitigen Ventil (V11) ausgerüstet ist, über welches öl- und staubfreie Luft über die Leitung (66) und hernach durch einen Drucksensor (PS1) und einen Durchflussmesser (FS1) führbar ist, dann durch eine $CO_2$- und $H_2O$-Abscheidekolonne (61), dann über einen zweiten Drucksensor (PS2) in ein $CO_2$-Analysegerät (62) und über ein anlageseitiges Ventil (V12) über eine Leitung (64) in den Stator-Kühlwassertank (2) einblasbar ist, und wobei das Steigrohr (13) vorhanden ist, von welchem mittels einer Pumpe (PM) mit ihrem Pumprad (63) Gas über ein anlagenseitiges Ventil (V13) abziehbar ist, das über ein $H_2$-Analysegerät und ein Ventil (14) in die Leitung (66) und über das Ventil (V12) zurück über die Leitung (64) in den Stator-Kühltank (2) führbar ist, und dass die Steuereinheit (65) eine elektronische Steuereinheit (25) mit Eingabefeld und Display ist, die als Kontroll-Box zur Anlage gehört, und mittels welcher die Messdaten aller angeschlossenen Sensoren und Analysegeräte der Anlage auswertbar sind, mindestens der $CO_2$-Gehalt der injizierten Luft und der $H_2$-Gehalt in diesem Steigrohr (13) messbar sind, und durch dieselbe bei Überschreiten eines eingebbaren Grenzwertes ein akustischer und optischer Alarm abgebbar ist.

2.  Anlage nach Anspruch 1, **dadurch gekennzeichnet, dass** mittels der elektronischen Steuereinheit (65) die Injektionsrate von Luft in das Stator-Kühlwasser-System über 0.15 cfm einhaltbar ist, dass die Drucksensoren (PS1) und (PS2) einen Messbereich von 0-100 psi aufweisen, zur Messung des Druckes in der angesaugten Luft, und mittels des Durchflussmesser (FS) ein Luftdurchfluss von 0,1-10 l/min messbar ist.

3.  Anlage nach einem der Ansprüche 1 bis 2, **dadurch gekennzeichnet, dass** mittels der elektronischen Steuereinheit (65) und des $CO_2$-Analysegerätes (62) der $CO_2$-Anteil in der eingeblasenen Luft in einem Messbereich einer $CO_2$-Konzentration von 1-1000 ppm messbar und anzeigbar ist, und bei einem frei wählbarem Wert ein akustischer und optischer Alarm abgebbar ist.

4.  Anlage nach einem der Ansprüche 1 bis 3, **dadurch gekennzeichnet, dass** sie an Stator-Kühlwassersystemen mit niedrigem Sauerstoffgehalt einsetzbar ohne $CO_2$-Entfernung ist, indem anstelle von Luft reiner Stickstoff $N_2$ in das Stator-Kühlwassersystemen einblasbar ist und die Wasserstoffleckage messbar und anzeigbar ist.

**5.** Anlage nach einem der Ansprüche 1 bis 4, **dadurch gekennzeichnet, dass** die elektronische Steuereinheit (65) eine App einschliesst, zum Versenden von akustischen Alarmen.

**6.** Anlage nach einem der Ansprüche 1 bis 5, **dadurch gekennzeichnet, dass** mittels der elektronischen Steuereinheit (65) alle von den angeschlossenen Messgeräten gemessenen Daten über Kabel oder drahtlos an einen oder mehrere CPU's auslesbar sind.

**Claims**

**1.** Installation for cooling cooling water-conducting copper pipes present in large electric generators cooled with hydrogen gas, with which installation, depending on the oxygen content of the cooling water, a minimum quantity of $CO_2$-free air can be pumped into the cooling water cooling circuit at a high oxygen content or pure nitrogen $N_2$ at a low oxygen content, and whereby the critical air quantity proportion for the hydrogen $H_2$ contained in the cooling circuit can be kept outside the range of 4-75% $H_2$ in the air contained in the cooling water and thus outside the dangerous quantity ratio, with a connection to a stator cooling water system, wherein the system comprises a stator cooling water tank (2) with outgoing pipe (3) and riser pipe (13), and wherein the outgoing pipe (3) leads the cooling water through pumps (4), heat exchangers (6), a filter (7) and thereafter optionally through an ion exchanger (9) back into the stator cooling water tank (2) or into the cooling channels of a stator winding in a generator (1), and wherein the installation includes an air injection system (60) with a control system, with measuring instruments for the continuous measurement of $CO_2$ in the incoming supply air and $H_2$ in the outgoing exhaust air, as well as a control unit (65) with operating elements, wherein limit values can be entered for the $CO_2$ concentration as well as for the $H_2$ concentration, the air injection system (60) being equipped with a valve (V11) on the plant side, via which oil- and dust-free air can be guided through the line (66) and then through a pressure sensor (PS1) and a flow meter (FS1), then through a $CO_2$ and $H_2O$ separation column (61), then via a second pressure sensor (PS2) into a $CO_2$ analyser (62) and via a plant-side valve (V12) via a line (64) into the stator cooling water tank (2), and wherein the riser pipe (13) is present, from which gas can be withdrawn via a plant-side valve (V13) by means of a pump (PM) with its pump impeller (63), which can be fed via an $H_2$ analyser and a valve (14) into the pipe (66) and via the valve (V12) back via the pipe (64) into the stator cooling tank (2), and in that the control unit (65) is an electronic control unit (25) with input field and display, which belongs to the system as a control box, and by means of which the measurement data of all connected sensors and analysis devices of the installation can be evaluated, at least the $CO_2$ content of the injected air and the $H_2$ content in this riser pipe (13) can be measured, and by means of which an acoustic and optical alarm can be emitted when an adjustable limit value is exceeded.

**2.** System according to claim 1, **characterised in that** by means of the electronic control unit (65) the injection rate of air into the stator-cooling water system can be kept above 0.15 cfm, that the pressure sensors (PS1) and (PS2) have a measuring range of 0-100 psi for measuring the pressure in the sucked-in air, and by means of the flow meter (FS) an air flow of 0.1-10 l/min can be measured.

**3.** System according to one of the claims 1 to 2, **characterised in that** by means of the electronic control unit (65) and the $CO_2$ analyser (62) the $CO_2$ content in the blown-in air can be measured and displayed in a measuring range of a $CO_2$ concentration of 1-1000 ppm, and at a freely selectable value an acoustic and optical alarm can be emitted.

**4.** System according to one of the claims 1 to 3, **characterised in that** it can be used on stator cooling water systems with low oxygen content without $CO_2$ removal, **in that** pure nitrogen $N_2$ can be injected into the stator cooling water system instead of air and the hydrogen leakage can be measured and displayed.

**5.** A system according to any one of claims 1 to 4, **characterised in that** the electronic control unit (65) includes an app for sending acoustic alarms.

**6.** System according to one of the claims 1 to 5, **characterised in that** by means of the electronic control unit (65) all data measured by the connected measuring devices can be read out via cable or wirelessly to one or more CPUs.

**Revendications**

**1.** Installation pour le refroidissement de conduites en cuivre conduisant l'eau de refroidissement, présentes dans de grands générateurs électriques refroidis à l'hydrogène gazeux, installation permettant de pomper dans le circuit de refroidissement de l'eau de refroidissement, en fonction de la teneur en oxygène de l'eau de refroidissement, une quantité minimale d'air exempt de $CO_2$ en cas de forte teneur en oxygène ou de l'azote pur $N_2$ en cas de faible teneur en oxygène, et grâce à laquelle la proportion critique d'air pour l'hydrogène $H_2$ contenu dans le circuit de refroidissement peut être maintenue en dehors de la plage de 4 à 75% de $H_2$ dans l'air contenu dans l'eau de refroidissement et donc en dehors du rapport quantitatif dangereux, avec un raccordement à un système stator/eau de refroidissement, l'installation comprenant un réservoir d'eau de refroidissement de stator (2) avec une conduite de sortie (3) et une colonne montante (13), et la conduite de sortie (3) ramenant l'eau de refroidissement à travers des pompes (4), un échangeur de chaleur (6), un filtre (7) et ensuite, au choix, à travers un échangeur d'ions (9) dans le réservoir d'eau de refroidissement de stator (2) ou dans les canaux de refroidissement d'un enroulement de stator dans un alternateur (1), et où l'installation comprend un système d'injection d'air (60) avec un système de contrôle, avec des instruments de mesure pour la mesure continue de $CO_2$ dans l'air amené et de $H_2$ dans l'air évacué, ainsi qu'une unité de commande (65) avec des éléments de commande, des valeurs limites pouvant être introduites pour la concentration de $CO_2$ ainsi que pour la concentration de $H_2$, en cas de dépassement de ces valeurs, une alarme peut être déclenchée ou des mesures peuvent être prises automatiquement, le système d'injection d'air (60) étant équipé d'une vanne (V11) côté installation, par laquelle de l'air exempt d'huile et de poussière peut être acheminé via la conduite (66) et ensuite via un capteur de pression (PS1) et un débitmètre (FS1), puis via une colonne de séparation de $CO_2$ et de $H_2O$ (61), puis, par l'intermédiaire d'un deuxième capteur de pression (PS2), dans un analyseur de $CO_2$ (62) et, par l'intermédiaire d'une vanne (V12) située du côté de l'installation, dans le réservoir d'eau de refroidissement (2) du stator par l'intermédiaire d'une conduite (64), et dans lequel est présent le tube ascendant (13) duquel du gaz peut être prélevé au moyen d'une pompe (PM) avec sa roue de pompe (63) par l'intermédiaire d'une vanne (V13) située du côté de l'installation, qui peut être amené dans la conduite (66) par l'intermédiaire d'un analyseur H2 et d'une vanne (14) et ramené dans le réservoir de refroidissement du stator (2) par la conduite (64) par l'intermédiaire de la vanne (V12), et en ce que l'unité de commande (65) est une unité de commande électronique (25) avec un champ de saisie et un écran, qui fait partie de l'installation en tant que boîtier de contrôle, et au moyen de laquelle les données de mesure de tous les capteurs et appareils d'analyse raccordés de l'installation peuvent être évaluées, au moins la teneur en $CO_2$ de l'air injecté et la teneur en $H_2$ dans cette colonne montante (13) peuvent être mesurées, et par laquelle une alarme acoustique et optique peut être émise en cas de dépassement d'une valeur limite pouvant être définie.

2. Installation selon la revendication 1, **caractérisée en ce que** l'unité de commande électronique (65) permet de maintenir le débit d'injection d'air dans le système d'eau de refroidissement du stator au-dessus de 0,15 cfm, **en ce que** les capteurs de pression (PS1) et (PS2) ont une plage de mesure de 0 à 100 psi pour mesurer la pression dans l'air aspiré, et **en ce que** le débitmètre (FS) permet de mesurer un débit d'air de 0,1 à 10 l/min.

3. Installation selon l'une des revendications 1 à 2, **caractérisée en ce qu'**au moyen de l'unité de commande électronique (65) et de l'appareil d'analyse de $CO_2$ (62), la proportion de $CO_2$ dans l'air insufflé peut être mesurée et affichée dans une plage de mesure d'une concentration en $CO_2$ de 1 à 1000 ppm, et une alarme acoustique et optique peut être émise pour une valeur pouvant être choisie librement.

4. Installation selon l'une des revendications 1 à 3, **caractérisée en ce qu'**elle peut être utilisée sur des systèmes d'eau de refroidissement de stator à faible teneur en oxygène sans élimination de $CO_2$, **en ce que** de l'azote pur N2 peut être injecté dans le système d'eau de refroidissement de stator à la place de l'air et **en ce que** la fuite d'hydrogène peut être mesurée et affichée.

5. Installation selon l'une des revendications 1 à 4, **caractérisée en ce que** l'unité de commande électronique (65) comprend une application pour l'envoi d'alarmes acoustiques.

6. Installation selon l'une des revendications 1 à 5, **caractérisée en ce que** l'unité de commande électronique (65) permet de lire toutes les données mesurées par les appareils de mesure raccordés, par câble ou sans fil, à une ou plusieurs unités centrales.

# Fig. 1

# Fig. 2

# Fig. 3

# Fig. 4

**IN DER BESCHREIBUNG AUFGEFÜHRTE DOKUMENTE**

**In der Beschreibung aufgeführte Patentdokumente**

- US 20070277593 A1
- JP H037040 A
- US 6459750 B1, ITO° KAZUTOSHI [JP]
- JP H05268750 A
- US 2007277593 A1, SALEM SAMEH [US]
- JP H0844081 A
- KR 101439523 B1
- US 2009260518 A1, WRIGHT ANDREW DAVID [GB]
- DE 102016106225 A1